# EUROPEAN PATENT APPLICATION

(11) **EP 4 729 013 A1**
(43) Date of publication of application: **22.04.2026**
(21) Application number: 24822851.2
(22) Date of filing: 17.06.2024
(51) Int. Cl.: A61B 34/20, A61B 34/30

(54) **REFERENCE FRAME FOR ELECTROMAGNETIC NAVIGATION DEVICE OF SURGICAL ROBOT, AND ELECTROMAGNETIC NAVIGATION DEVICE**

(30) Priority: 16.06.2023 CN 202310717945; 16.06.2023 CN 202321552596 U
(71) Applicant: CHUNFENGHUAYU (SUZHOU) INTELLIGENT MEDICAL TECHNOLOGY CO., LTD., Taicang Suzhou, Jiangsu 215413 (CN)
(72) Inventor: YOUNG, Victor, Suzhou, Jiangsu 215413 (CN); CHEN, Chaoliang, Suzhou, Jiangsu 215413 (CN); SHI, Weisong, Suzhou, Jiangsu 215413 (CN)
(74) Representative: Maiwald GmbH
(86) International application number: PCT/CN2024/099582
(87) International publication number: WO 2024/255904

(57) **Abstract**

The present application relates to a reference frame for electromagnetic navigation of a surgical robot, the surgical robot having a robotic arm. The reference frame comprises: a reference frame body; a holder, the holder being operably connected to the reference frame body, and drivable, so as to fixedly maintain the reference frame body on a rigid tissue closest to a target area; a drive member used to drive the holder. An accommodation portion, used to detachably mount an electromagnetic sensing device used for electromagnetic navigation, and multiple visible bodies, capable of displaying coordinate positions in imaging device images, are arranged on the reference frame body.

## Description

### CROSS-REFERENCE TO RELATED APPLICATION

For all purposes, the present disclosure claims priority to Chinese Application No. 202310717945.4 filed on June 16, 2023 and Chinese Application No. 202321552596.7 filed on June 16, 2023, which are incorporated herein by reference in their entirety.

### TECHNICAL FIELD

The present disclosure relates to a reference frame for electromagnetic navigation of a surgical robot with a robotic arm. Furthermore, the present disclosure also relates to an electromagnetic navigation device for a surgical robot and an electromagnetic navigation system.

### BACKGROUND

Navigation devices for surgical robots are used to track the position of one or more objects during surgery. Surgical robots are suitable for holding one or more surgical instruments during surgery and can operate autonomously (e.g., without any manual input during surgery), semi-autonomously (e.g., with some manual input during surgery), or non-autonomously (e.g., only as directed by manual input).

Currently, surgical robot systems are commonly used in surgical operations, which typically include structures such as a cart, a robotic arm arranged on the cart, an optical navigation device, an imaging device, an operating table, etc, the patient undergoes surgery on the operating table, and the navigation device can determine the pose (position and orientation) of one or more target bodies during surgery to detect movement of the target bodies, such as movement of the robotic arm, movement of surgical tools, movement of patient anatomy, etc. To accurately determine the pose of a target body, it is usually necessary to set up a reference frame on the target body, a plurality of reflective balls are provided on the body of this reference frame and can be used to collect reference coordinate information of the target body during surgery and direct the robotic arm to work. In existing surgical robot systems navigation is achieved by registration of imaging device coordinates, infrared optical positioning coordinates, and surgical instrument coordinates through infrared optical navigation devices for navigation, this infrared navigation device is easily obstructed by objects during surgery, thereby the navigation effects of such optical navigation-based surgical robot systems can be adversely affected.

### SUMMARY

The basic technical problem to be solved by the present disclosure is to provide an improved reference frame for an electromagnetic navigation device for a surgical robot, an electromagnetic navigation device with this reference frame, and an electromagnetic navigation system, which can avoid the drawback of the optical navigation of surgical robots being easily obstructed by objects in the prior art and achieve high-precision navigation.

According to a first aspect of the present disclosure, the above technical problem is solved by a reference frame for electromagnetic navigation of a surgical robot with a robotic arm, according to the first aspect of the present disclosure, the reference frame comprises: a reference frame body; a holding member operatively connected to the reference frame body and capable of being driven to fixedly hold the reference frame body on rigid tissue closest to a target area; a drive member for driving the holding member; wherein a receiving part for detachably mounting an electromagnetic sensing device for the electromagnetic navigation is provided on the reference frame, particularly on its reference frame body, and a plurality of markers capable of displaying coordinate positions in images of an imaging device are arranged on the reference frame, particularly on its reference frame body. By fixing both the electromagnetic sensing device and the markers on the same component, namely the reference frame, particularly its reference frame body, it is possible to ensure that the relative position between the electromagnetic sensing device and the markers is fixed, thereby ensuring that the accuracy of the electromagnetic navigation for the surgical robot is sufficiently high. Alternatively, the electromagnetic sensing device and the markers can also be arranged on the holding member or the drive member.

In a further technical solution of the present disclosure, the reference frame body comprises a distal wall portion away from the rigid tissue during surgery and leg portions extending from the distal wall portion towards the rigid tissue. The distal wall portion enables the holding member and the reference frame body to be connected to each other, thereby reliably transmitting the driving force from the drive rod, thus ensuring that the reference frame can be stably held on the rigid tissue.

In a further embodiment of the present disclosure, the reference frame body further comprises a plurality of web portions connected between the leg portions, thereby facilitating the machining of holes for mounting the joint shaft. Preferably, two opposing webs can be provided, thereby ensuring that the holding member does not move in the longitudinal direction of the reference frame body, thus more effectively transmitting the holding force used to hold the reference frame on the rigid tissue.

In a further embodiment of the present disclosure, the reference frame body at least partially accommodates the holding member, particularly such that the holding member can freely transition between its different operational states, thus ensuring that the operational state of the holding member can be easily changed, especially ensuring that the holding member can freely transition between its holding state and its open state.

In a further embodiment of the present disclosure, the plurality of markers are respectively provided on the proximal end regions of the leg portions. In particular, the number of markers can be set differently according to application requirements.

In a further embodiment of the present disclosure, the holding member has at least two holding arms and a pivot shaft, around which the at least two holding arms are hinged. This allows the reference frame to be conveniently held on the rigid tissue closest to the surgical site of the patient, while ensuring that the driving force is advantageously converted into a holding force.

In a further embodiment of the present disclosure, a plurality of through holes are provided in the reference frame body, the plurality of through holes being arranged on different parts of the reference frame body. Alternatively, the plurality of through holes can also be provided on the drive rod or the holding member. By providing a plurality of through holes on different parts of the reference frame body, such as the distal wall portion and the leg portions, it is ensured that the different components of the reference frame and the electromagnetic sensing device can be coupled to each other and facilitate mechanical processing and assembly.

In a further embodiment of the present disclosure, one of the plurality of through holes is a first through hole for passing through the drive member, and two of the plurality of through holes are second through holes for mounting the pivot shaft, wherein the first through hole and each of the second through holes are oriented perpendicular to each other. This allows the various parts of the reference frame body to be coupled to each other in an advantageous manner, especially coupling the drive member and the holding member to the reference frame body in an advantageous manner, while achieving the conversion of the driving force into the holding force for holding the reference frame on the rigid tissue in an advantageous way.

In a further embodiment of the present disclosure, the first through hole has an internal thread and is provided in the distal wall portion, and the second through holes are provided in the web portions. This facilitates the mechanical machining of the through holes into the reference frame body and also makes assembly of the reference frame easier.

In a further embodiment of the present disclosure, the drive member comprises a screw and a boss, the screw is provided with a distal section, a middle section, and a proximal section, wherein a limiting device for restricting longitudinal movement of the boss along the screw is provided on the proximal section, and an external thread is provided on the middle section, the drive member engaging with the internal thread of the first through hole via the external thread.

In a further embodiment of the present disclosure, the limiting device comprises a stop near the external thread and a retaining ring far from the external thread, the retaining ring being spaced apart from the stop along the longitudinal direction of the screw.

In a further embodiment of the present disclosure, the stop protrudes radially outward from the outer periphery of the screw. The stop can be designed as annular or arc-shaped.

In a further embodiment of the present disclosure, the distal section of the drive member is designed with a polygonal external cross-section, which can be rotated by a screwing tool.

In a further embodiment of the present disclosure, the at least two holding arms each have a distal end and a proximal end, the distal end contacting the drive member when driven by the drive member, and the proximal end contacting the rigid tissue when holding the rigid tissue.

In a further embodiment of the present disclosure, a plurality of opposing pointed tips are respectively provided on the inner sides of the proximal ends of the at least two holding arms.

In a further embodiment of the present disclosure, the receiving part is designed as a hole with an internal thread for threaded connection with the electromagnetic sensing device.

In a further embodiment of the present disclosure, a calibration coordinate point for imaging device image registration is also provided on the reference frame body.

According to another aspect of the present disclosure, the aforementioned technical problem is solved by an electromagnetic navigation device for a surgical robot with a robotic arm. According to the present disclosure, the electromagnetic navigation device comprises: the aforementioned reference frame; a plurality of electromagnetic sensing devices, one of which is a first electromagnetic sensing device for sensing the real-time pose of the rigid tissue, detachably mounted on the reference frame, and another of which is a second electromagnetic sensing device for sensing the real-time pose of an end effector of the robotic arm, fixed on the robotic arm; an electronic control unit configured to control a magnetic field generating device to generate a magnetic field to obtain the position of a first electromagnetic sensor arranged on the reference frame; a magnetic field generating device configured to generate a magnetic field covering the first sensing device and the second sensing device above a target area to be operated on; an imaging device configured to perform intraoperative scanning of the target area and the reference frame body to obtain coordinate positions of the target area and the markers on the reference frame based on an imaging device coordinate system.

In a further technical solution of the present disclosure, the first electromagnetic sensing device has a connector with an external thread for detachably fixing to the reference frame, a first electromagnetic sensor, and a cable, through which the first electromagnetic sensor on the reference frame is coupled to the electronic control unit, the first electromagnetic sensor being arranged in the connector.

According to another aspect of the present disclosure, the aforementioned technical problem is solved by an electromagnetic navigation system for a surgical robot. According to the present disclosure, the electromagnetic navigation system has: the aforementioned electromagnetic navigation device; a cart device for fixing the robotic arm relative to a target object and for placing the electronic control unit of the electromagnetic navigation device; a display device for displaying intraoperative imaging results and providing an operation interface; a robotic arm for guiding the movement of the end effector, fixedly connected to the cart device; an end effector for performing surgical operations on the target area, fixedly connected to the proximal end of the robotic arm.

More details and advantages of the examples described in detail herein will become clearer from the following detailed description of examples with reference to the accompanying drawings.

### BRIEF DESCRIPTION OF DRAWINGS

Fig. 1 is a schematic diagram of an electromagnetic navigation system for a surgical robot during spinal surgery according to a non-limiting embodiment of the present disclosure;
Fig. 2 is a schematic diagram of a local area A related to the reference frame in Fig. 1;
Fig. 3 is a view of the reference frame held on a spinous process as observed from the right side of Fig. 2;
Fig. 4 is a view of the end region of the first electromagnetic sensing device connected to the reference frame body;
Fig. 5 is a perspective schematic diagram of a reference frame body according to a non-limiting embodiment of the present disclosure;
Fig. 6 is a perspective view of a drive member of a reference frame according to a non-limiting embodiment of the present disclosure;
Fig. 7 is a perspective view of a holding member of a reference frame according to a non-limiting embodiment of the present disclosure;
Fig. 8 is a longitudinal sectional view of the reference frame in Fig. 3 in a holding state;
Fig. 9 is a longitudinal sectional view of the reference frame in Fig. 3 in an open state.

Referring to the drawings, wherein the same reference numbers refer to the same components in the several views, the present disclosure generally relates to a reference frame for electromagnetic navigation of a surgical robot, an electromagnetic navigation device for a surgical robot, and an electromagnetic navigation system for a surgical robot.

### DETAILED DESCRIPTION

It should also be understood that the specific apparatuses, devices, and systems shown in the drawings and described in the following specification are merely exemplary illustrations of the present disclosure. Therefore, specific dimensions and other physical characteristics related to the examples disclosed herein should not be considered limiting.

When the term "distal" is used relative to components of the reference frame, electromagnetic navigation device, and system for a surgical robot (such as the holding member, screw, reference frame body), this term "distal" refers to the side of the component farthest from the patient. When the term "proximal" is used relative to components of the reference frame, electromagnetic navigation device, and system for a surgical robot (such as the holding member, screw, reference frame body), this term "proximal" refers to the side of the component closest to the patient. When the term "distal end" is used relative to components of the reference frame, electromagnetic navigation device, and system for a surgical robot (such as the holding member, screw, reference frame body), this term "distal end" refers to the end region of the component farthest from the patient. When the term "proximal end" is used relative to components of the reference frame, electromagnetic navigation device, and system for a surgical robot (such as the holding member, screw, reference frame body), this term "proximal end" refers to the end region of the component closest to the patient. Furthermore, within the scope of the present disclosure, the term "target object" refers to a patient to be operated on by the surgical robot, the term "target area" refers to the patient's body part involved in the surgical operation performed by the surgical instrument, and the term "holding object" refers to the rigid tissue near the aforementioned body part, particularly bone tissue such as a spinous process. The directional terms "above", "below", "left", and "right" are used with respect to the corresponding drawings of the present disclosure. The terms "first", "second" merely indicate that they are not the same component and do not indicate a limitation of order.

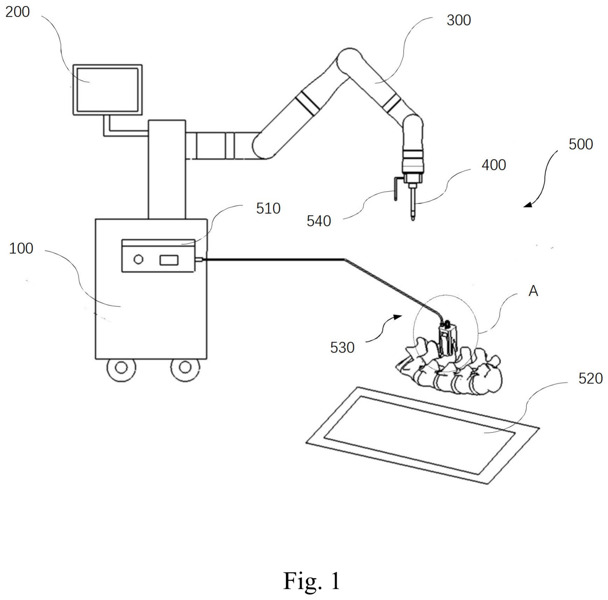

Fig. 1 is a schematic diagram of an electromagnetic navigation system for a surgical robot during spinal surgery according to a non-limiting embodiment of the present disclosure. It includes a cart device 100, a display device 200, a robotic arm 300, an end effector 400, and an electromagnetic navigation device 500 for a surgical robot, which is particularly used to achieve intraoperative navigation. The robotic arm 300 is preferably designed as a multi-axis robotic arm in the present disclosure. One end of the robotic arm 300 can be fixed to the cart 100, and the other end of the robotic arm 300 can fix the end effector 400 and is used to guide the movement of the end effector 400. The cart device 100 is configured to fix the robotic arm 300 relative to the target object, i.e., the patient to be operated on, and to place the electronic control unit 510. As an alternative, the cart device 100 can also fix the robotic arm 300 relative to the operating table. This cart device is also preferably equipped with lockable rollers to make the operation of the robotic surgical system of the present disclosure more flexible. The display device 200 is configured to display the imaging results of the intraoperative imaging device to the surgeon and to provide an operation interface. The display device can be designed, for example, as a liquid crystal display. The end effector 400 is a surgical instrument for performing surgical operations on the target area, in a non-limiting embodiment, it is a spinal surgical instrument, for example, a screw placement tool, a drilling tool, etc., which is fixedly connected to the proximal end of the robotic arm 300. The end effector 400 can be detachably mounted on the robotic arm 300 via a multi-purpose surgical instrument connector. The electromagnetic navigation device 500 for a surgical robot, as a core component of the electromagnetic navigation system, is primarily responsible for the real-time positioning and navigation of the target area and the end effector 400 during surgery. This electromagnetic navigation device 500 will be further described below.

In a non-limiting embodiment, the electromagnetic navigation device 500 for a surgical robot comprises a reference frame 550, a plurality of electromagnetic sensing devices, an electronic control unit 510, a magnetic field generating device 520, and an imaging device (not shown in the Figs. ), particularly a three-dimensional imaging device, one of which is a first electromagnetic sensing device 530 for sensing rigid tissue, such as a spinous process, and thereby obtaining the real-time pose of the target area based on its relative positional relationship with the target area to be operated on, the first electromagnetic sensing device 530 is detachably mounted on the reference frame 550 (which will be further elaborated below), and another electromagnetic sensing device is a second electromagnetic sensing device 540 for sensing the real-time pose of the end effector 400 of the robotic arm 300, which is fixed to the proximal end region of the robotic arm 300, the fixation can particularly be done via a surgical instrument connector. The electronic control unit 510 can control the magnetic field generating device 520 to generate a magnetic field, especially a three-dimensional magnetic field, to obtain the position of the first electromagnetic sensor arranged on the reference frame 550. The magnetic field generating device 520 can generate a magnetic field above the target area to be operated on, covering the (not shown) first electromagnetic sensor on the reference frame 550 and the second electromagnetic sensor on the robotic arm. The imaging device can perform intraoperative scanning, especially three-dimensional scanning, of the target area and the reference frame 550 to obtain the coordinate positions, especially the three-dimensional coordinate positions, of the target area and the markers 5514 on the reference frame 550 (to be further elaborated below) based on the imaging device coordinate system. In a non-limiting embodiment, this imaging device is preferably designed as a CT scanner.

The operating principle of the electromagnetic navigation device according to the present disclosure is preferably, but not limited to: first, performing an intraoperative three-dimensional CT scan of the target area and the reference frame 550 with a CT scanner (not shown) to obtain the coordinate positions of the target area and the markers 5514 on the reference frame 550 based on the CT coordinate system; next, the electronic control unit 510 controls the magnetic field emitting device 520 to generate a three-dimensional magnetic field to obtain the position of the first electromagnetic sensor on the reference frame 550; since the relative position between the markers 5514 on the reference frame 550 and the first electromagnetic sensor on the reference frame 550 is known, the CT coordinates and the electromagnetic field coordinates can be matched according to this relative positional relationship; additionally, the positional relationship between the second electromagnetic sensor on the robotic arm 300 and the free end of the end effector is known, and through this positional relationship, the electromagnetic field coordinates and the coordinates of the robotic arm 300 can be matched; finally, the registration of the CT, electromagnetic field, and robotic arm 300 coordinates is achieved.

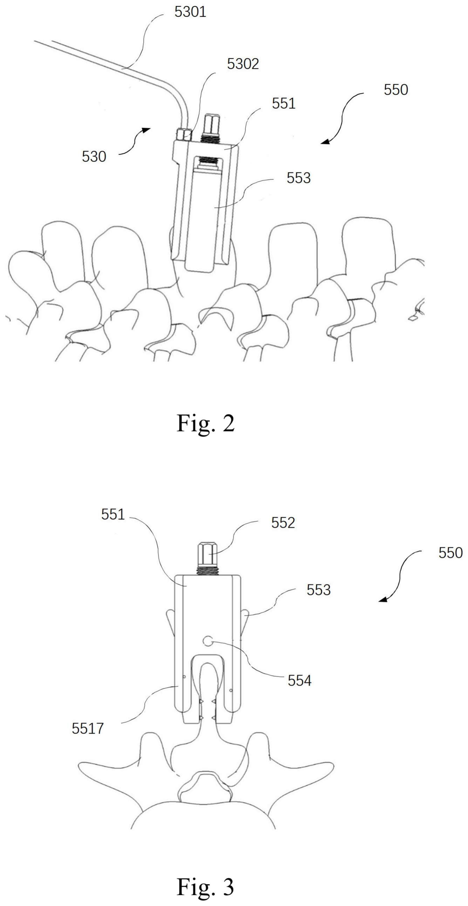

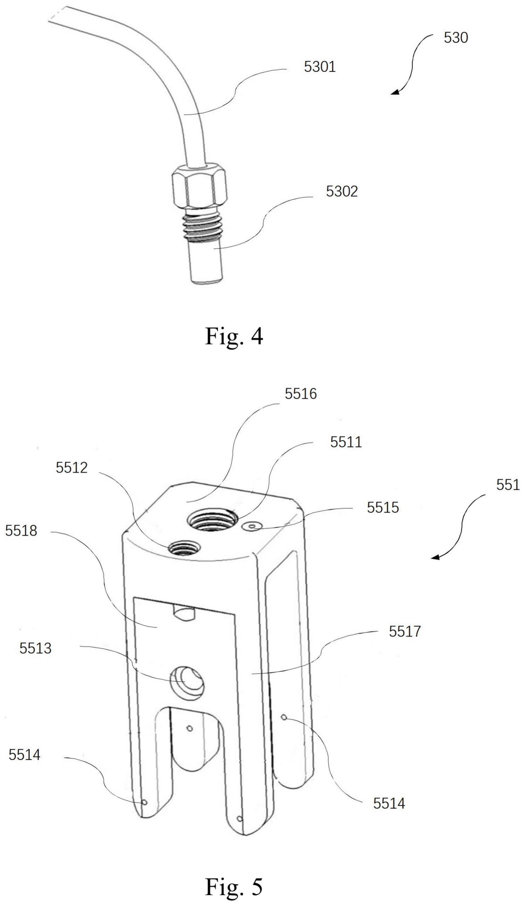

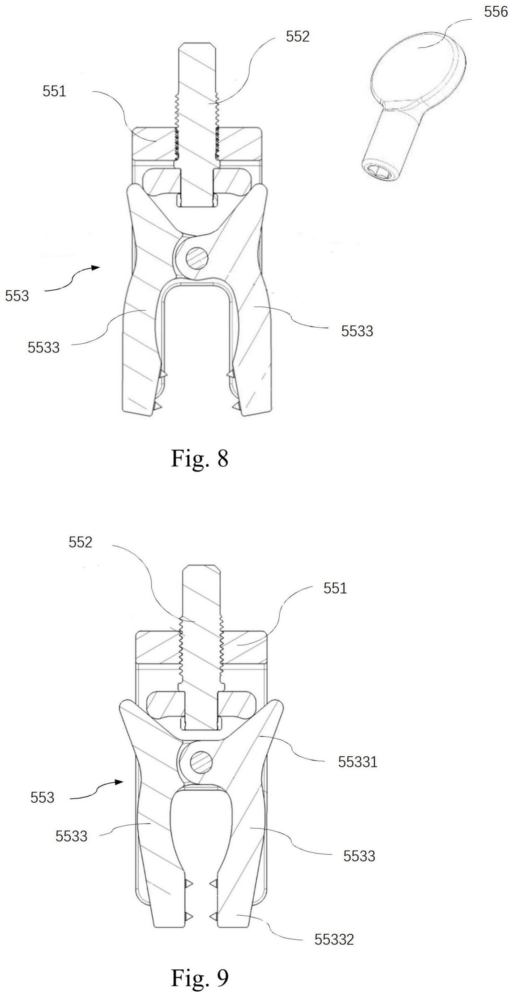

Figs. 2 and 3 are views of the local area A related to the reference frame 550 in Figure 1 from different perspectives. They show the structure of different sides of the reference frame 550, an exemplary assembly situation of the first electromagnetic sensing device 530 with the reference frame 550, and an example of the reference frame 550 fixed on a spinous process during surgery. As shown in Fig. 2, the first electromagnetic sensing device 530 (to be further elaborated below) can be fixed to the reference frame 550, particularly to its reference frame body's distal wall portion 5516, to facilitate the assembly of the first electromagnetic sensing device onto the reference frame. The proximal end of this first electromagnetic sensing device 530 is coupled to the reference frame 550 via a connector 5302, and the distal end of the first electromagnetic sensing device 530 is coupled to the electronic control unit 510 shown in Fig. 1, in order to transmit the data collected by the electromagnetic sensor to the electronic control unit 510. In a non-limiting embodiment, the reference frame 550 can have a holding member 553 for holding the reference frame 550 on rigid tissue, a drive member 552 for driving the holding member 553, and a reference frame body 551 for coupling the various parts of the reference frame 550. The reference frame body 551 at least partially accommodates the holding member 553, such that the holding member 553, when driven, can assume different operational states within the gaps between the leg portions 5517, especially the open state and the holding state shown in Figs. 8 and 9. To fix the reference frame more stably and securely on the rigid tissue, the leg portions can be designed such that when the leg portions 5517 are in the holding state as shown in Fig. 3, i.e., when the leg portions 5517 embrace the rigid tissue, the proximal end faces of at least two of the leg portions 5517 contact the rigid tissue to support on the corresponding rigid tissue. In one non-limiting embodiment, the holding member 553 protrudes at least partially from the sides of the reference frame body, or in other words, from the left and right sides in Fig. 3, beyond the reference frame body 551, especially protruding from the gaps between its leg portions 5517 (see also Fig. 5). Depending on different application scenarios during surgery, the holding member 553 can also be designed in the form of a clamping member as in Fig. 3 or in the form of a spreading member not shown here. In the case where the holding member 553 is designed as a spreading member (not illustrated in the Figs. to highlight key content), the reference frame 550 can be fixed on the spinous process by means of the two holding arms 5533 of the holding member 553 being supported between two corresponding spinous processes.

Fig. 4 is a view of the end region of the first electromagnetic sensing device 530 connected to the reference frame body 551, especially a view of the proximal end region of the first electromagnetic sensing device 530, showing the exemplary external structure of the first electromagnetic sensing device 530. In a non-limiting embodiment, this first electromagnetic sensing device 530 has a cable 5301, a connector 5302, and a first electromagnetic sensor (not visible in the Figs.) for sensing the real-time pose of the target area. This first electromagnetic sensor is preferably arranged within or on the connector 5302. Alternatively, the first electromagnetic sensor can also be arranged at other locations of the electromagnetic sensing device, as long as it does not affect the assembly of the electromagnetic sensing device onto the reference frame and is sufficiently secure. The connector 5302 can be provided with external threads or a snap-fit structure. To conveniently connect or fix the first electromagnetic sensing device to the reference frame 550, especially the reference frame body 551, the external threads shown in Fig. 4 can be made to engage with the internal threads of the through hole 5511 of the reference frame body 551 shown in Fig. 5 by screwing the connector 5302, or the first sensing device can be inserted into a through hole (not shown in Fig. 5) of the reference frame body 551 to allow an alternative snap-fit structure to interlock with a corresponding snap-fit structure of the through hole (not shown in Fig. 5) of the reference frame body 551.

Fig. 5 is a perspective view of the reference frame body 551 according to a non-limiting embodiment of the present disclosure. The structure of the reference frame body 551 is clearly visible from this figure. In one non-limiting embodiment, the reference frame body 551 can comprise a distal wall portion 5516 away from the rigid tissue during surgery and leg portions 5517 extending from the distal wall portion 5516 towards the rigid tissue. Additionally or alternatively, the reference frame body 551 further comprises a plurality of web portions 5518 connected between the leg portions 5517. As a supplement or alternative, two web portions 5518 can be provided, which can preferably be opposed to each other, to facilitate the installation of the (to be further elaborated) pivot shaft 5532 of the holding member 553. Of course, in another non-limiting embodiment, the reference frame body 551 may not have web portions; in this case, the pivot shaft 5532 can be mounted on the leg portions 5517, especially on two opposing leg portions. A plurality of through holes can be provided on the reference frame body 551, and the plurality of through holes can be arranged on different parts of the reference frame body 551. As a supplement or alternative, one of the plurality of through holes is a first through hole 5511 for passing the drive member 552 through, and two of the plurality of through holes are second through holes 5513 for mounting the pivot shaft 5532. As a supplement or alternative, the first through hole 5511 and the second through holes 5513 are oriented perpendicular to each other, to facilitate the easy installation of the holding member 553 onto the reference frame body 551 and to enable the holding member 553 to reliably transition between operational states within the reference frame body 551. To facilitate assembly and mechanical processing between the components of the reference frame 550, the first through hole 5511 has an internal thread and is provided in the distal wall portion 5516 of the reference frame body 551, and the second through holes 5513 are provided in the web portions 5518 of the reference frame body 551. Alternatively, the first through hole and the second through holes can also be provided in other parts of the reference frame body 551, as long as they are perpendicularly oriented. A calibration coordinate point 5515 for imaging device image registration can also be mounted on the reference frame body 551. This calibration coordinate point 5515 is particularly configured for determining the corresponding positions of the markers 5514 and the electromagnetic sensor. In one non-limiting embodiment, this calibration coordinate point 5515 is provided on the distal wall portion 5516 of the reference frame body 551. Alternatively, the calibration coordinate point 5515 can also be provided at other locations on the reference frame, especially the reference frame body, that are convenient for installation and/or processing. As a supplement or alternative, a plurality of markers 5514 are arranged in the proximal end regions of the leg portions 5517 of the reference frame body 551. The plurality of markers 5514 can be displayed under the imaging device, especially display coordinate positions in the imaging device's images, in order to register with the images taken by the three-dimensional imaging device of the electromagnetic navigation device during surgery.

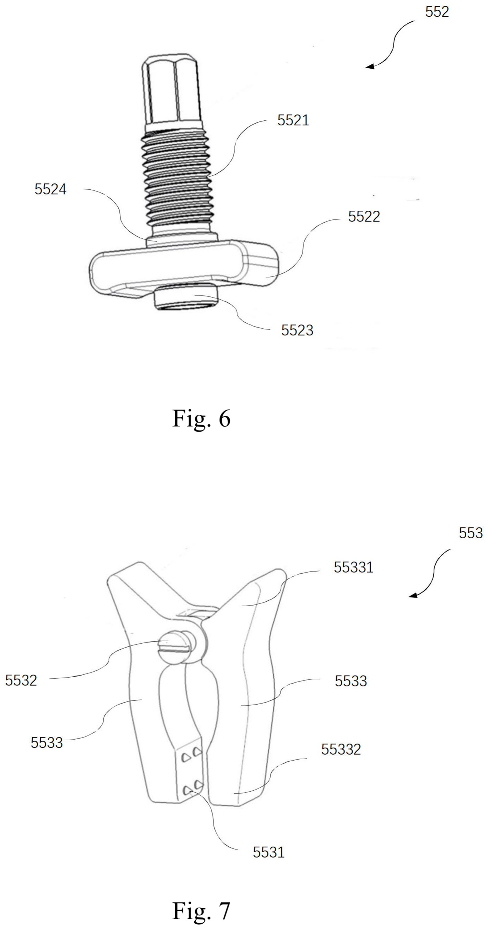

Figure 6 is a perspective view of the drive member 552 of the reference frame 550 according to a non-limiting embodiment of the present disclosure. In one non-limiting embodiment, the drive member 552 comprises a screw 5521 and a boss 5522. The screw 5521 has a distal section, a middle section, and a proximal section. The boss 5522 can be designed to contact the distal region of the holding member 553 when driving the holding member 553. As a supplement or alternative, the boss can be fixedly mounted on the screw 5521, or it can be freely rotatably mounted on the screw 5521, and a limiting device for restricting the longitudinal movement of the boss 5522 along the screw 5521 is provided on the proximal section of the screw 5521. In one non-limiting embodiment, an external thread is provided on the middle section. The drive member 552 engages with the internal thread of the first through hole 5511 of the reference frame body 551 via the external thread, so that the drive member 552 is movably connected to the reference frame body 551. To facilitate the application of external force to securely hold the reference frame 550 on the rigid tissue, it is preferable that the distal section of the drive member 552 is designed with a polygonal external cross-section, so that it can be rotated by a screwing tool 556. As a supplement or alternative, the boss 5522 is arranged on the proximal section of the drive member 552 to contact the distal end of the holding member 553. To restrict the longitudinal movement of the boss 5522 along the screw 5521, it is preferable that the limiting device comprises a stop 5524 near the external thread of the screw 5521 and a retaining ring 5523 far from the external thread, wherein the retaining ring 5523 is spaced apart from the stop 5524 along the longitudinal direction of the screw 5521. In one non-limiting embodiment, the stop 5524 protrudes radially outward from the outer periphery of the screw 5521, wherein the stop can be designed as annular or arc-shaped.

Figure 7 is a perspective view of the holding member 553 of the reference frame 550 according to a non-limiting embodiment of the present disclosure. The holding member 553 has at least two holding arms 5533 for holding the rigid tissue and a pivot shaft 5532. The at least two holding arms 5533 are hinged around the pivot shaft 5532. The at least two holding arms 5533 respectively have a distal end 55331 and a proximal end 55332. The distal end 55331 of the holding member 553 contacts the drive member 552 when driven by the drive member 552, and the proximal end 55332 contacts the rigid tissue when holding the rigid tissue. A plurality of opposing pointed tips 5531 are respectively provided on the inner sides of the proximal ends 55332 of the holding arms 5533, to enable the reference frame 550 to be held more securely on the rigid tissue, especially to clamp or support more firmly on the rigid tissue.

Figs. 8 and 9 are longitudinal sectional views of the reference frame 550 in Figure 3 in different operational states, respectively. Figure 8 shows the reference frame 550 with its holding member 553 in the holding state, and Figure 9 shows the reference frame 550 with its holding member 553 in the open state. The process of detachably fixing the reference frame 550 onto the rigid tissue is exemplarily explained below with reference to these two Figs.: By screwing the drive member 552 with a screwing tool 556 whose internal cross-section corresponds to the external cross-section of the distal end of the drive member 552, the drive member 552 is moved relative to the reference frame body 551 towards the bottom of the figure. At this time, since the holding member 553 is rotatably fixed to the reference frame body 551 via the pivot shaft 5532 and therefore cannot move in the vertical direction of the figure, as the drive member 552 moves downward, the distal ends 55331 of the two holding arms 5533 of the holding member 553 are pressed downward relative to the figure by the drive member 552, especially its boss 5522. The holding arms 5533 subsequently pivot around the pivot shaft 5532, thereby causing the proximal ends 55332 of the holding member 553 to tightly embrace the rigid tissue (as in Figure 9). Conversely, when the holding member 553 is fixedly held on the rigid tissue, by screwing the drive member 552 in the opposite direction with the screwing tool 556, the drive member 552 is moved away from the holding member 553. As the boss leaves the distal ends 55331 of the two holding arms 5533, the proximal ends of the holding member 553 gradually open and thereby release the rigid tissue (as in Figure 8).

Although various examples of the present disclosure have been provided in the foregoing description, those skilled in the art can make modifications and alterations to these examples without departing from the scope and spirit of the present disclosure. For example, it should be understood that features of the various embodiments described herein can be applicable to other embodiments described here. Therefore, the description is intended to be illustrative rather than limiting. The disclosure is defined by the following claims, and all amendments to the disclosure that fall within the meaning and range of equivalency of the claims are intended to be embraced within their scope.

## Claims

1. A reference frame for electromagnetic navigation of a surgical robot with a robotic arm, **characterized in that** the reference frame comprises:
a reference frame body;
a holding member, the holding member being operatively connected to the reference frame body and capable of being driven to fixedly hold the reference frame body on rigid tissue closest to a target area;
a drive member for driving the holding member;
wherein a receiving part for detachably mounting an electromagnetic sensing device for the electromagnetic navigation is provided on the reference frame, and a plurality of markers capable of displaying coordinate positions in images of an imaging device are arranged on the reference frame.

2. The reference frame according to claim 1, **characterized in that** the reference frame body comprises a distal wall portion away from the rigid tissue during surgery and leg portions extending from the distal wall portion towards the rigid tissue.

3. The reference frame according to claim 2, **characterized in that** the reference frame body further comprises a plurality of web portions connected between the leg portions.

4. The reference frame according to any one of claims 1 to 3, **characterized in that** the reference frame body (551) at least partially accommodates the holding member.

5. The reference frame according to claim 2 or 3, **characterized in that** the plurality of markers are respectively provided on proximal end regions of the leg portions.

6. The reference frame according to any one of claims 1 to 3, **characterized in that** the holding member has at least two holding arms for holding the rigid tissue and a pivot shaft, wherein the at least two holding arms are hinged around the pivot shaft.

7. The reference frame according to claim 3, **characterized in that** a plurality of through holes are provided in the reference frame body, wherein the plurality of through holes are arranged in different parts of the reference frame body.

8. The reference frame according to claim 7, **characterized in that** one of the plurality of through holes is a first through hole for passing through the drive member, and two of the plurality of through holes are second through holes for mounting the pivot shaft, wherein the first through hole and each of the second through holes are oriented perpendicular to each other.

9. The reference frame according to claim 8, **characterized in that** the first through hole has an internal thread and is provided in the distal wall portion, and the second through holes are provided in the web portions.

10. The reference frame according to claim 9, **characterized in that** the drive member comprises a screw and a boss, the screw is provided with a distal section, a middle section, and a proximal section, wherein a limiting device for restricting longitudinal movement of the boss along the screw is provided on the proximal section, and an external thread is provided on the middle section, wherein the drive member engages with the internal thread of the first through hole via the external thread.

11. The reference frame according to claim 10, **characterized in that** the limiting device comprises a stop near the external thread and a retaining ring far from the external thread, wherein the retaining ring is spaced apart from the stop along a longitudinal direction of the screw.

12. The reference frame according to claim 11, **characterized in that** the stop protrudes radially outward from an outer periphery of the screw.

13. The reference frame according to claim 10, **characterized in that** the distal section of the drive member (552) is designed with a polygonal external cross-section, which can be rotated by a screwing tool (556).

14. The reference frame according to claim 6, **characterized in that** the at least two holding arms (5533) each have a distal end and a proximal end, wherein the distal end contacts the drive member when driven by the drive member, and the proximal end contacts the rigid tissue when holding the rigid tissue.

15. The reference frame according to claim 14, **characterized in that** a plurality of opposing pointed tips are respectively provided on inner sides of the proximal ends of the at least two holding arms (5533).

16. The reference frame according to any one of claims 1 to 3, **characterized in that** the receiving part (5512) is designed as a hole with an internal thread for threaded connection with the electromagnetic sensing device.

17. The reference frame according to any one of claims 1 to 3, **characterized in that** a calibration coordinate point (5515) for imaging device image registration is also provided on the reference frame body.

18. An electromagnetic navigation device for a surgical robot with a robotic arm, **characterized in that** the electromagnetic navigation device comprises:
the reference frame according to any one of claims 1 to 17;
a plurality of electromagnetic sensing devices, one of which is a first electromagnetic sensing device for sensing a real-time pose of the rigid tissue, detachably mounted on the reference frame, and another of which is a second electromagnetic sensing device for sensing a real-time pose of an end effector (400) of the robotic arm, fixed on the robotic arm;
an electronic control unit (510) configured to control a magnetic field generating device (520) to generate a magnetic field to obtain a position of a first electromagnetic sensor arranged on the reference frame (550);
a magnetic field generating device (520) configured to generate a magnetic field covering the first sensing device (530) and the second sensing device (540) above a target area to be operated on;
an imaging device configured to perform intraoperative scanning of the target area and the reference frame body to obtain coordinate positions of the target area and the markers on the reference frame based on an imaging device coordinate system.

19. The electromagnetic navigation device according to claim 18, **characterized in that** the first electromagnetic sensing device has a connector (5302) with an external thread for detachably fixing to the reference frame, a first electromagnetic sensor, and a cable (5301), through which the first electromagnetic sensor on the reference frame is coupled to the electronic control unit (510), wherein the first electromagnetic sensor is arranged in the connector (5302).

20. An electromagnetic navigation system for a surgical robot, wherein the electromagnetic navigation system has:
the electromagnetic navigation device (500) according to claim 18 or 19;
a cart device (100) for fixing the robotic arm (300) relative to a target object and for placing the electronic control unit (510) of the electromagnetic navigation device;
a display device (200) for displaying intraoperative imaging results and providing an operation interface;
a robotic arm (300) for guiding movement of an end effector (400), fixedly connected to the cart device (100);
an end effector (400) for performing surgical operations on the target area, fixedly connected to a proximal end of the robotic arm (300).
